# EUROPEAN PATENT SPECIFICATION

(11) **EP 2 456 402 B1**
(45) Date of publication and mention of the grant of the patent: **20.08.2014**
(21) Application number: 10802768.1
(22) Date of filing: 20.07.2010
(51) Int. Cl.: A61F 9/009, A61F 9/008

(54) **LIQUID HOLDING INTERFACE DEVICE FOR OPHTHALMIC LASER PROCEDURES**
FLÜSSIGKEITSHALTENDE SCHNITTSTELLENVORRICHTUNG FÜR OPHTHALMISCHE LASER-VORGÄNGE
DISPOSITIF D'INTERFACE CONTENANT UN LIQUIDE POUR PROCÉDURES LASER OPHTALMIQUES

(30) Priority: 24.07.2009 US 228457 P
(43) Date of publication of application: 30.05.2012
(73) Proprietor: Lensar, Inc., Orlando, FL 32826 (US)
(72) Inventor: PORTER, Gerrit, N., Winter Springs FL 32708 (US); FREY, Rudolph, W., Winter Park Florida 32789 (US); BOTT, Steven, E., Oviedo FL 32765 (US); OLMSTEAD, Richard, Ty, Oviedo FL 32765 (US); BUNCH, Theodora, Jane, Winter Park Oviedo FL 32792 (US)
(74) Representative: Boult Wade Tennant
(86) International application number: PCT/US2010/042582
(87) International publication number: WO 2011/011400

(56) References cited:
- EP-A1- 1 970 034
- WO-A1-03/002010
- CA-A1- 2 680 072
- US-A- 3 074 407
- US-A- 4 334 736
- US-A- 4 744 362
- US-A- 5 616 139
- US-A1- 2002 029 053
- US-A1- 2004 249 403
- US-A1- 2006 192 921
- US-A1- 2007 093 795
- US-A1- 2009 187 178

## Description

### BACKGROUND OF THE INVENTION

### Field of the Invention

The present invention relates to an apparatus for use in laser surgery on the eye and in particular during cataract laser surgery. Thus, the present invention provides for a fluid holding patient interface device that does not obstruct the laser beam from contacting the limbus region of the cornea.

### Discussion of Related Art

In performing laser surgery on the eye, and in particular in performing laser surgery on the lens of the eye, such as for the purpose of performing a laser assisted capsulotomy, an interface device is needed that touches the anterior surface of the eye and docks with the laser device that is being used to perform the surgery. Such laser devices and systems are disclosed, for example, in published applications US 2007/173794 A1, US 2007/173795 A1, US 2007/185475 A1, WO 2007/084694 A2, WO 2007/084627 A2.

A particular problem that may occur with prior interface devices arises because the laser beam must pass through the interface device which has a different index of refraction than the components of the eye that the laser beam must also pass through to reach the lens. This difference in the index of refraction creates difficulties in accurately placing the laser beam at a particular point in the lens of the eye or at particular points in other structures within the eye. Previously, this problem has been addressed by the use of complex mathematical algorithms that attempted to compensate for the difference in the index of refraction. Moreover, this problem is even further increased when the surfaces of the interface device through which the laser beam must travel are not flat. A further limitation of such devices is that the structures of the devices may block or otherwise interfere with the ability of the laser beam from the laser device to cut the limbus.

### SUMMARY

It is desirable to develop a device that reduces or eliminates these and other undesirable features of such devices. In particular it is desirable to have a device that has one or more of the following features: it is adjustable with respect to the variations in the cornea; it has the same index of refraction as the cornea; it does not applanate the cornea; and, it does not interfere with delivery of the laser beam or access by biometric devices used to measure lens structures, to the cornea, limbus, sclera and anterior and posterior surfaces of the crystalline lens.

The present invention, among other things, solves this need and provides these features by providing a self-adjusting liquid holding interface device for use with a laser surgery apparatus. The interface device contains a reference glass plate suitable for registering the position of the laser surgery apparatus to that of lens structures such as the anterior and posterior cornea and crystalline lens surfaces during measurements of lens structure by biometric devices associated with the laser surgery apparatus. The device is configured in the shape of a ring shaped structure, the ring shaped structure having a distal end and a proximal end, the distal end intended to be positioned toward the eye and the proximal end intended to be positioned toward the laser device, the ring shaped structure having a length that is measured by the distance between the distal and proximal ends. The ring shaped structure having an outer annular structure and an inner annular structure, the outer annular structure and the inner annular structure are connected or associated with one another in a slidable manner that enables telescoping movement between them. The ring shaped structure having a vacuum chamber positioned on its distal end for engaging the surface of an eye; and having a fluid reservoir.

A further embodiment of the present invention provides that the device has a first vacuum chamber that is positioned on the distal end of the outer annular structure, a second vacuum chamber that is positioned on the distal end of the inner annular structure; and, the first and second vacuum chambers are not in fluid communication, whereby the device can apply different amounts of vacuum through the first and second vacuum chambers.

The forgoing and following embodiments of the present invention further may be filled with an index matching solution, such as saline. Means are also provided to prevent the entrapment of bubbles within the index matching solution reservoir during the filling operation, to prevent obscuration of the laser beam by such bubbles.

There is still further provided a self-adjusting interface device for use with a laser surgery apparatus including a structure, the structure having a distal end and a proximal.end, the structure having a length measured by the distance between the distal and proximal ends. The structure having an outer structure and an inner structure, the outer and inner structures having distal and proximal ends, the outer structure and the inner structure adapted so as to provide the capability for their respective distal ends to move relative to each other and thereby adapt to varying curvatures of an eye. The inner and outer structures each having their own independent vacuum chamber positioned on the distal end for engaging the surface of an eye.

There is additionally provided an adjusting liquid holding interface device for use with a laser surgery apparatus, including: a structure, the structure having a distal end and a proximal end, the structure having a length measured by the distance between the distal and proximal ends; the structure having an outer structure and an inner structure; the outer and inner structures having distal and proximal ends; the distal end of the structure having a vacuum chamber for attaching to the eye; and, the structure configured to provide the capability for a therapeutic laser beam to cut the limbus region of the eye while the device is affixed to the eye by the vacuum chamber.

The forgoing and following embodiments of the present invention may have one or many vacuum chambers for attaching and holding the device and its movable parts to the eye. This chamber may be in fluid communication with each other, in which case all such chambers will apply the same level of vacuum. Alternatively, some of the chambers may not be in fluid communication with other chambers, in which case such chambers will have the capability to apply different levels of vacuum and doing so to different sections of the eye.

### BRIEF DESCRIPTION OF THE DRAWINGS

FIG. 1 is a sectional diagram of an embodiment of a device in accordance with the present invention when positioned on an eye.
FIG. 2 is a top planar view of a diagram of the device of FIG. 1.
FIGS. 3-8 are views of a second embodiment of a device in accordance with the present invention.
FIG. 9 is a top, side perspective and exploded view of a third embodiment of a device in accordance with the present invention.
FIG. 10 is a bottom, side perspective and exploded view of the device of FIG. 9.
FIG. 11 is a right side view of the device of FIG. 9.
FIG. 12 is a bottom view of the device of FIG. 9.
FIG. 13 is a front view of the device of FIG. 9.
FIG. 14 is a top, side perspective view of the device of FIG. 9.

### DESCRIPTION OF THE DRAWINGS AND THE PREFERRED EMBODIMENTS

The present invention provides for a laser patient interface device that is fluid holding, and can be filled with a fluid that preferably matches the index of refraction of the anterior chamber of the eye and thus greatly reduces any difference in the index of refraction between the eye and the interface device.

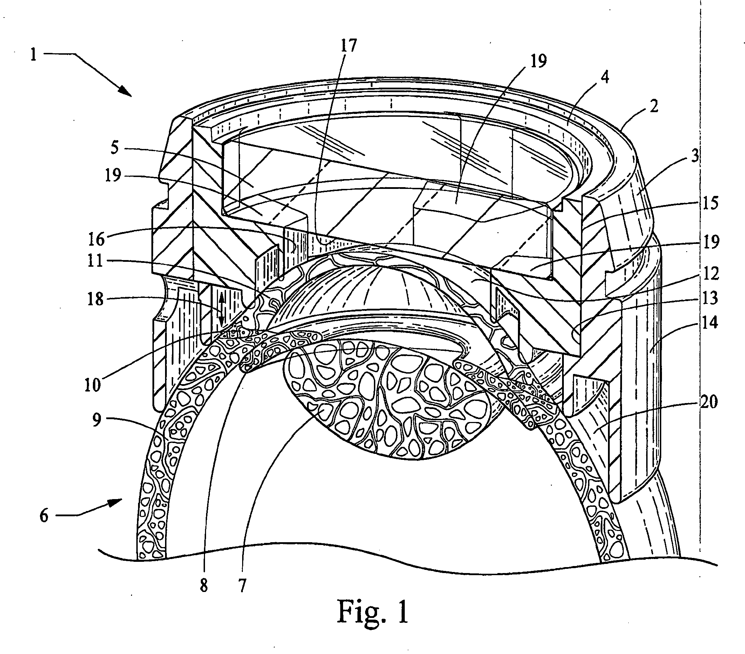

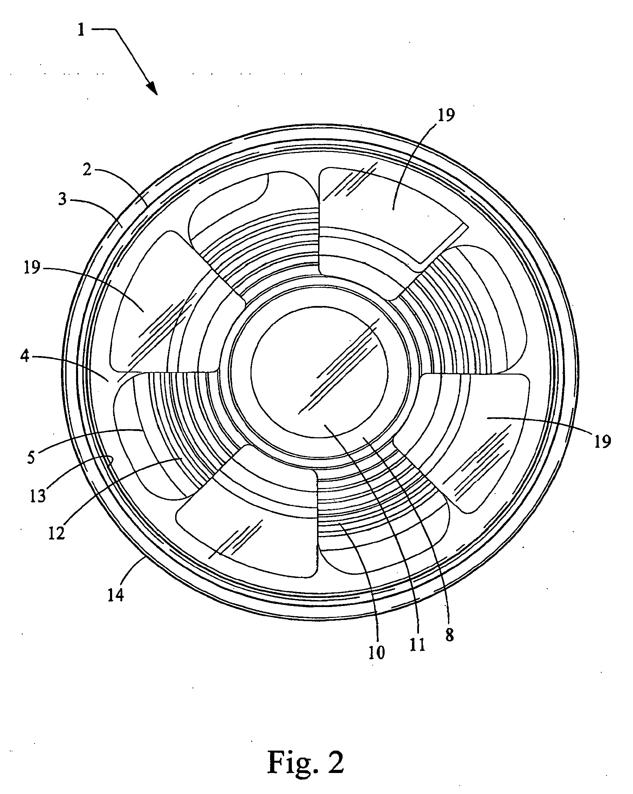

In general, as shown in FIGS. 1 - 2 there is provided a liquid holding interface device 1 for use in the performance of ophthalmic laser surgery. The device 1 has a distal or bottom (as illustrated and positioned in the drawings) end, which engages the eye, and a proximal or top (as illustrated and positioned in the drawings) end, which is disposed toward the laser apparatus. Thus, there is provided, by way of example, a device 1 having a ring shaped structure 2. The ring shaped structure 2 has an outer structure 3, an inner structure 4, and a glass plate 5. The outer structure 3 has an inner surface 13 and an outer surface 14. The inner structure 4 has an outer surface 15 and an inner surface 16. The outer surface 15 is adjacent to the inner surface 13.

As seen in the figures, the device 1 is placed on an eye 6. The relative size and position of the device 1 on the eye 6 is shown with respect to the lens 7, iris 8, sclera 9, limbus 10 and cornea 11.

A fluid reservoir 12 is formed by the bottom 17 of the glass plate 5 and the inner surface(s) of the ring structure(s). Thus, as seen in the drawings a fluid reservoir is formed by the bottom 17 of glass plate 5 and the inner surface 13 of the outer structure 3. In a variation of this device the inner surface of the inner ring could be configured to a part of the fluid reservoir. In a further variation, the inner and outer structures are one and the inner surface of that structure forms a portion of the reservoir. The components of the reservoir are connected together in a manner that is fluid tight. The reservoir is then held in place on the eye, and rendered fluid tight with the eye, while on the eye in the orientation shown in the drawings, by suction that is applied to vacuum chambers as described in greater detail herein. The device 1 has a filling means or port, not shown, which can be used to fill the reservoir 12 of the device 1 with a liquid. The device and its structures may be made from plastic, glass and/or metal.

The reservoir 12, when positioned on the eye and after suction has been applied, can be filled with a fluid having a known index of refraction and thus the index of refraction can be set to match and/or approximate the index of refraction of the lens of the eye 6. Thus, the reservoir is preferably filled with a balanced salt solution ("BSS") or saline solution that has been degassed. Moreover, although the preferred embodiment of the present invention is to match or as closely as possible approximate the index of refractions of the device to that of the eye, in other applications having known and predetermined difference may be advantageous. Thus, the reservoir may be filled with a particular index matching fluid having a predetermined and known index of refraction, such as those that are obtainable from NYE and CARGILLE LABS.

The glass plate 5 is as thin as practicable and may be about 0.5 mm to 2 mm in thickness. The glass plate may have a diameter of about 11 mm to 16 mm. The diameter is chosen to be as small as will allow the therapeutic laser to reach the cornea and limbus and allow any associated biometric devices requiring access to the eye through the glass plate to have the required access. Such biometric devices might be required to measure sizes, shapes and positions of various eye structures such as the anterior and posterior surfaces of the cornea and crystalline lens. The glass plate may be affixed to the device by glue, pressure fit or other techniques known to the art that would provide a fluid tight seal. Alternatively, a thin gap may be present between the glass plate and the device to allow air and bubbles to be expelled from the fluid reservoir 12 during the process of filling the reservoir with fluid so that such bubbles are not trapped in the fluid reservoir where they might obscure or deflect the therapeutic laser beam.

In the preferred embodiment of the interface device, the inner structure 4 and the outer structure 3 are associated with one another in a manner that permits the movement of the inner structure with respect to the outer structure as shown by arrow 18. In this way the outer structure may be positioned and affixed to the sclera, with the inner structure 4 and glass plate 5 having the ability to move up and down in a telescopic manner to accommodate the height of the cornea. In this way the device can self-adjust for patient-to-patient and eye-to-eye differences in the structure and shape of eyes. Thus, the device can attach to any eye without flattening or materially changing the shape of the cornea.

The preferred embodiment of the interface device further has a plurality of vacuum chambers that are capable of applying suction to the eye. For example, the inner structure 4 can have four vacuum chambers 19. These chambers may be in fluid communication with each other, and thus a common vacuum source may be used to apply suction to these chambers and further provide that the amount of suction is equal across all four vacuum chambers. The outer structure 3 can have a single annular vacuum chamber 20. This vacuum chamber is positioned to attach to the sclera. Thus, the device provides for different levels of vacuum to be applied to the cornea and the sclera. The pressure levels with the plurality of vacuum chambers can be varied over the course of the processes of affixing the interface device to the eye, mating of the inner to outer structures during docking and applying the therapeutic laser pulses after docking. The varying of the pressure allows the interface device to be firmly held when needed to prevent loss of fixation of the device to the eye, for example at times when lateral forces may be applied to the interface device during docking, and lower suction pressure to be applied, for example after docking, to minimize the period of time during which elevation of the eye's intraocular pressure by the interface device is sufficient to cause vision to black out due to blockage of blood flow to the optic nerve.

The device 1 is connected to a docking apparatus for connection to the laser surgery device. The device 1 may be associated with the docking apparatus as an integral or separable part. The shape of this docking apparatus is dependent upon and should match with the docking features of the laser surgery device. An example of such a docking apparatus is disclosed in U.S. Patent Application Serial No. 12/509,021, published as US 2010/0022994. The device 1 may thus be adapted for attachment to a docking apparatus.

In use, the device 1 is placed upon the eye and the eye is centered with respect to the laser beam path. Once centered, a vacuum is applied to chamber 20, and the device is affixed to the eye in the centered position. After the chamber 20 is affixed to the eye, a vacuum is then applied to chambers 19. This vacuum pulls the inner structure 4 and glass plate 5 and fixes it to the eye and fixes its relationship to the outer structure 3. Preferably once the chambers 19 are placed under vacuum and more preferably after the vacuum has been applied to both chambers, the reservoir 12 is filled with a liquid and the laser procedure can begin. In this manner the centration of the eye is accomplished with the aid of the patient, as the power of the cornea is available for focusing. After centration and fixation of the eye, the index matching fluid can be added, thus eliminating the power of the cornea for the purposes of directing the laser to the structures of the eye.

The shape and positions of the structures of the device enable the laser to be used for all cutting procedures that take place in a cataract surgery. Thus, the device 1 does not interfere with, permits and assists the laser to be used for creating the initial, and any subsequent incisions, into the limbus of the eye for the insertion of instruments and tools that are used in the cataract surgery. This result is achieved because the inner structure does not completely block the limbus and sclera regions of the eye from the laser beam. The device also permits and assists in the use of the laser to perform a capsulotomy and to section the opacified lens for removal from the lens capsule and replacement with a lens filling material.

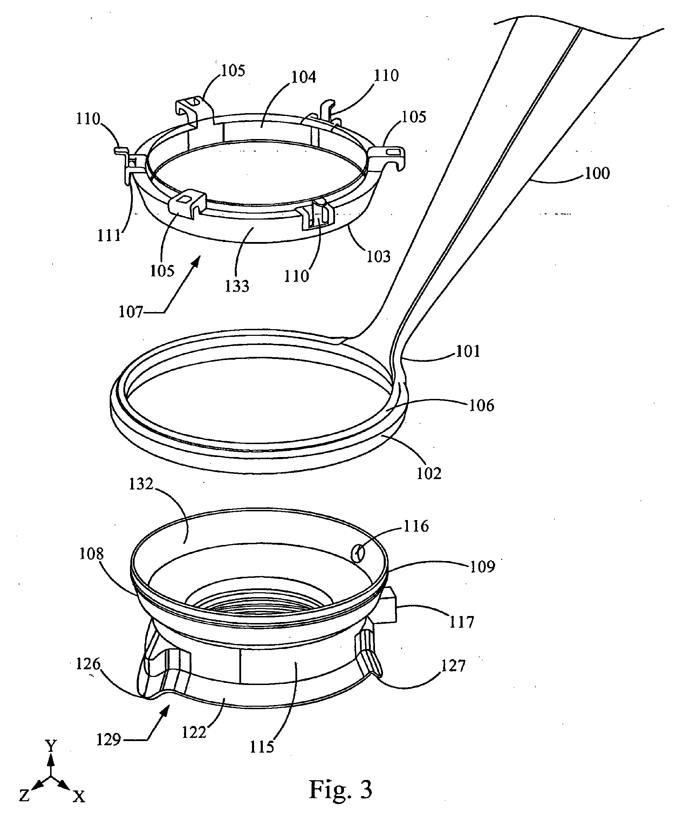

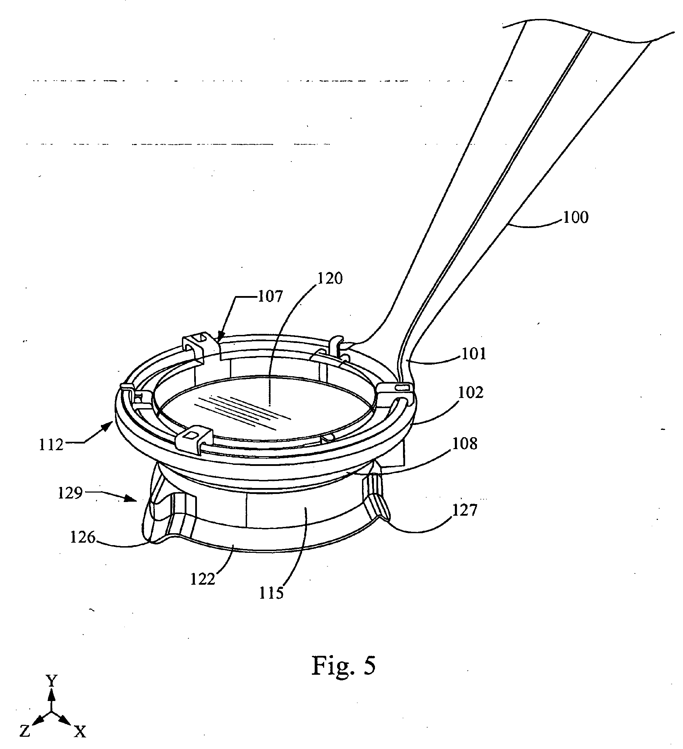

There is further provided a fluid holding patient interface docking device having in general a laser-patient interface member, a glass plate that is positioned in association with the laser-patient interface member, and a suction and fluid holding device that is joinable to the glass plate and interface member. Thus, by way of example as illustrated in FIGS. 3 to 8 there is provided a patient interface (like numbers in these figures refer to like components). Thus, the patient interface includes an arm 100. The arm has an upper end that is connect to the laser device (not shown in the figures) and a lower end 101 that is connected to a ring 102. The arm and ring are preferably made of a unitary material that is reusable and can be sterilized in a doctor's office, such as by use of an autoclave. However, the arm and/or the ring may be made of different materials that are disposable, not autoclaveable, and which are not unitary but may be fixedly and/or removably connected together, as well as combinations of such materials.

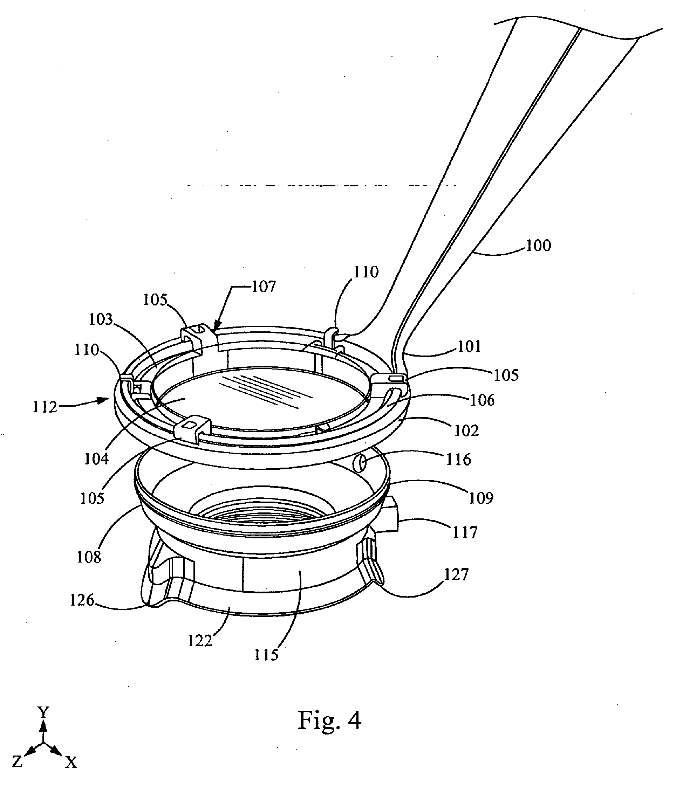

The device further has an adapter ring 103 for holding a glass plate 104. The adapter ring 103 has clips 105 and 110. The clips are designed to attach to and hold the adapter ring 103 in the ring 102. Preferably, and by way of example, the clips 105 are configured to mate with a lip 106 on ring 102. In this way, as illustrated for example in FIGS. 3 and 4, the glass plate 104 is attached to the adapter ring 103, preferably by gluing. A thin gap between glass plate 104 and adapter ring 103 allows air and bubbles expelled during the filling of the liquid holding chamber 120 to escape and to avoid obscuration of the therapeutic laser beam by entrapped bubbles. The proximal surface of the glass plate 104 is anterior to the proximal surface of the adapter ring 103 so that fluid overflowing the liquid holder chamber 120 during filling will not pool on the glass plate and obscure or divert the therapeutic laser beam. The adapter ring 103 and glass plate 104 assembly 107 is then clipped, e.g., fixedly removably attached, to the ring 102 of the arm 100. The glass plate 104 provides a reference surface for biometry devices associated with the surgical laser device to allow precise measurements of the location of lens structures, such as the anterior and posterior cornea and crystalline lens, with respect to the surgical laser device. In use, preferably the ring-glass assembly 107 would be provided to the surgeon for use as a sterile disposable part of a kit. The ring-glass assembly 107 could then be affixed into the ring 102 for use with a patient and disposed of after such use. The glass-ring assembly 107 when clipped to the ring 102 forms an upper assembly 112, that in use functions, among other things, as the upper part of a liquid holding chamber 120.

A housing 108 is provided with a mating lip 109 for mating to the lower portion 111 of clips 110. In this way the housing 108 can be fixedly removably attached to the ring 102 by means of the clips 110. The connection between the housing 108 and the ring 103 of the glass-ring assembly 107 is essentially a fluid tight seal. In the preferred embodiment this is accomplished by a pressure fit between the outer surface 133 of adaptor ring 103 and the inner surface 132 of housing 108. The housing 108 is preferably made of a stiff plastic material, or a metal, such as aluminum, which is sterilizable. The housing 108 has a lower lip 113 that mates to a corresponding mating lip 114 on suction ring 115.

The-housing 108 has a fluid port 116 for adding and removing fluid from the chamber 120. The fluid port may further contain or have associated therewith valves, tubing and suitable fluid deliver components to add, hold and remove fluid from the chamber. The suction ring 115 has a vacuum port 117 for removing air, i.e., creating a reduced pressure zone, from the suction ring lower pressure chamber 118 to hold suction ring 115 to the eye 130. The suction ring 115 is preferably made from a flexible material that will support the creation of a holding suction, such as silicone. The suction ring 115 is further joined to the housing 108 by gluing or bonding such that an essentially fluid tight seal is formed. In this way the housing 108 and the suction ring 115 form the sides of the liquid holding chamber 120. The assembly of suction ring 115 and housing 108 forms a lower assembly 129.

As used throughout the present application, the term "essentially fluid tight" seal refers to a seal that holds the liquid in the liquid chamber under its intended conditions of use and during the intended duration of that use, i.e., it holds the liquid during a laser surgery and related procedures.

The suction ring 115 has two annular suction members 121 and 122 and annular suction member 122 has three ridges 123, 124 and 125. In this way member 121 and member 122 (primarily ridges 123, 124 and 125 for member 122) touch the surface of the eye. In general it is preferable for inner member 121 to be located on or just outside of the limbus. Thus, with the members touching the eye a low pressure, or partial vacuum sufficient to hold the ring in place, can be created in the lower pressure chamber 118. Further, member 121 provides a fluid tight seal against the eye, which enables liquid holding chamber 120 to be associated with the eye in a manner that will enable the chamber to hold the fluid. The suction ring 115 may further have ears 126 and 127, which assist in the placement and holding of the ring to the eye.

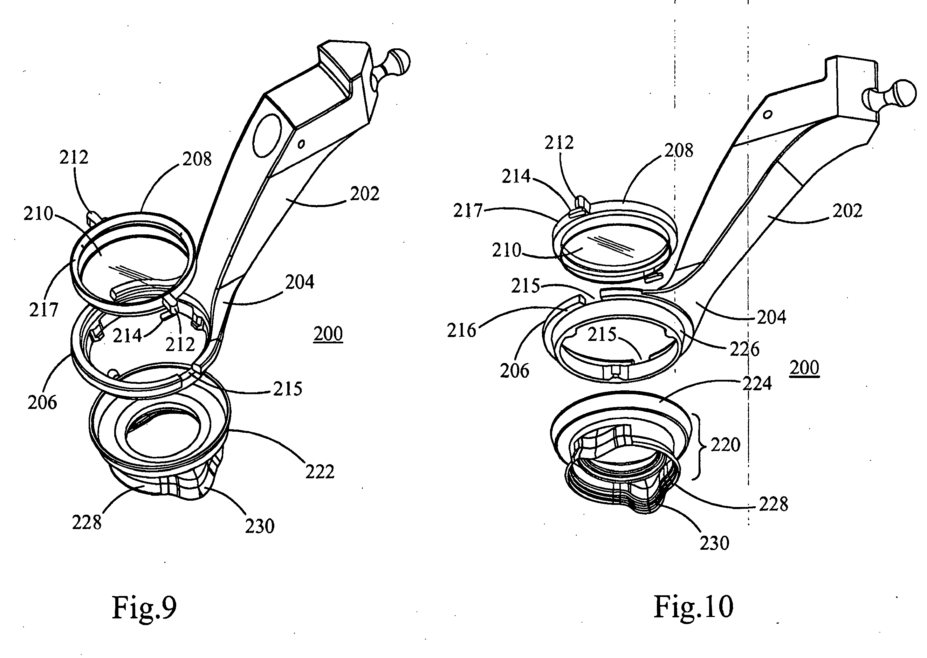

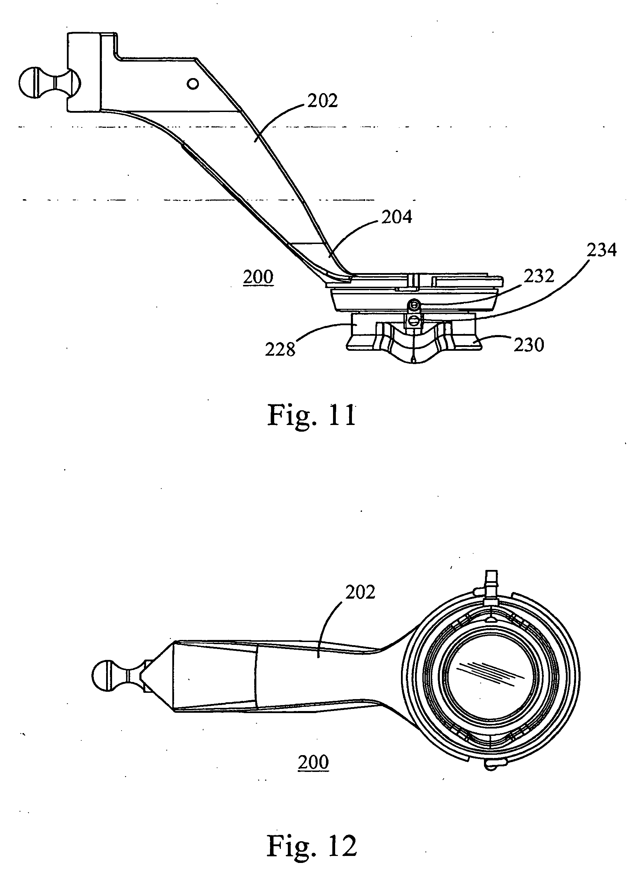

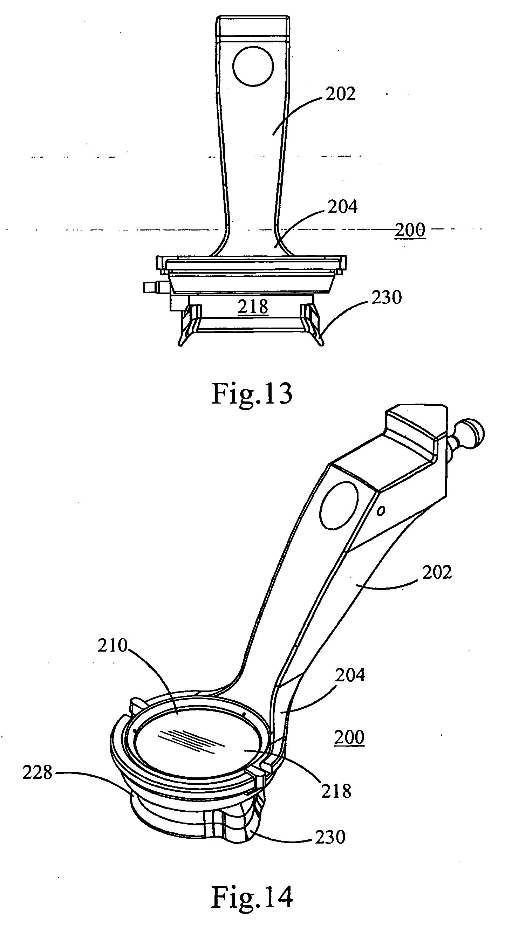

A third embodiment of a liquid holding interface device for use in the performance of ophthalmic laser surgery is shown in FIGS. 9-14. Operation of the liquid holding interface device 200 of FIGS. 9-14 is similar to that described in the embodiments of FIGS. 1-8. The device 200 includes an arm 202 that has an upper end that connects to the laser device (not shown in the figures) and a lower end 204 that includes a ring 206. The arm 202 and ring 206 are preferably made of a unitary material that is reusable and can be sterilized in a doctor's office, such as by use of an autoclave. However, the arm 202 and/or the ring 206 may be made of different materials that are disposable, not autoclaveable, and which are not unitary but may be fixedly and/or removably connected together, as well as combinations of such materials.

The device further has an upper assembly 217 of a liquid holding chamber 218, wherein the upper assembly includes an adapter ring 208 for holding a glass or fused silica plate 210. The plate 210 is attached to the adapter ring 208 via gluing, for example. The adapter ring 208 has a pair of oppositely positioned male extensions 212 that include ramped surfaces 214. The extensions 212 are designed to attach to and hold the adapter ring 208 in the ring 206. Preferably, and by way of example, the extensions 212 are inserted into corresponding slots 215 formed in the ring 206. Then, the adapter ring 208 is rotated so that the ramped surfaces 214 engage the underside of the lip 216 of the ring 206 so that a sufficient frictional attachment between the ring 206 and adapter ring 208 is achieved. Removal of the upper assembly 217 defined by plate 210 and ring 208 is accomplished by rotating the ring 208 in a direction opposite to the rotational direction that accomplished attachment.

When positioned for use, the plate 210 can be used as a reference surface for biometry devices associated with the surgical laser device to allow precise measurements of the location of lens structures, such as the anterior and posterior cornea and crystalline lens, with respect to the surgical laser device. Alternatively, the ring 206 can be used to precisely hold a calibration fixture, used for calibrating the system; the calibration fixture would be removed and replaced with glass plate 210 for the biometry. In this case, the plate 210 would not be used as a reference surface. In use, preferably the upper assembly 217 would be provided to the surgeon for use as a sterile disposable part of a kit. The upper assembly 217 could then be affixed into the ring 206 for use with a patient and disposed of after such use.

The device 200 further includes a lower assembly 220 of the liquid holding chamber 218. The lower assembly includes a housing 222 that is provided with a conical-like mating lip 224 for mating to a lower conical-like portion 226 of the ring 206. In operation, when the lower assembly 220 is positioned on the eye, the arm 202, with ring 206 and upper assembly 217 attached thereto, is lowered. During this lowering, the portion 226 is inserted into the mating lip 224 of housing 222. Combined with the constant downward force (approximately 113 to 340 g (4 to 12 ounces)) of the arm 202 and the close pressure fit between the portion 226 and the mating lip 224, the connection between the housing 222 and the ring 206 is essentially a fluid tight seal. Disconnection between the housing 222 and ring 206 is accomplished by raising the arm 202 and manually removing the two elements from one another. The housing 222 is preferably made of a stiff plastic material, or a metal, such as aluminum, which is sterilizable. The housing 222 mates to a suction ring 228 in a manner similar to the housing and suction ring of FIGS. 3-8. The suction ring 228 is preferably made from a flexible material that will support the creation of a holding suction, such as silicone. The suction ring 228 is further joined to the housing 222 by gluing, bonding or a tight fit between the slightly elastic suction ring 228 and the housing 222, such that an essentially fluid tight seal is formed. In this way the housing 222 and the suction ring 228 form the sides of the liquid holding chamber 218. The assembly of suction ring 228 and housing 222 forms a lower assembly 220.

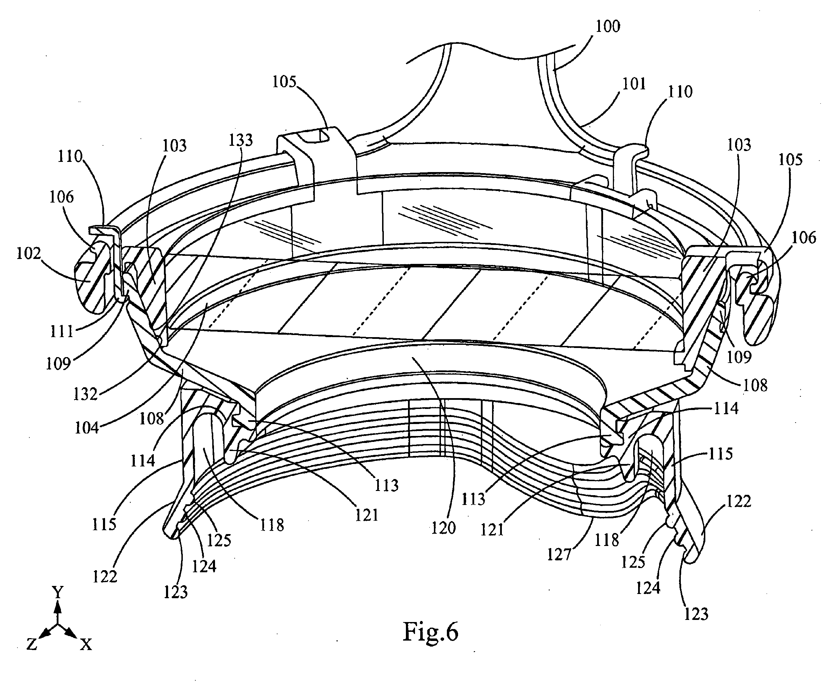

The suction ring 228 is similar to the suction ring 115 of FIGS. 3-8 and includes two annular suction members: an inner annular suction member like suction member 121 of FIG. 6 and an outer annular suction member 230. The outer annular suction member 230 has three ridges like ridges 123, 124 and 125 of FIG. 6. In this way the inner annular suction member and the three ridges of outer annular suction member 230 touch the surface of the eye. In general it is preferable for inner annular suction member to be located on or just outside of the limbus. Thus, with the inner and outer annular suction members touching the eye a vacuum chamber is formed therebetween. When a low pressure or partial vacuum is applied to the vacuum chamber, the ring 206 is held in place on the eye. In addition, the inner annular suction member provides a fluid tight seal against the eye, which enables liquid holding chamber 218 to be associated with the eye in a manner that will enable the chamber to hold the fluid.

The housing 222 has a fluid port 232 for adding and removing fluid from the chamber 218. The fluid port 232 may further contain or have associated therewith valves, tubing and suitable fluid delivery components to add, hold and remove fluid from the chamber 218. The lower assembly 220 has a vacuum port 234 for removing air, i.e., creating a reduced pressure zone, from the vacuum chamber to hold the lower assembly 220 to the eye. In contrast to the embodiment of FIGS. 3-8, only one vacuum port is used in the embodiment of FIGS. 9-14. Of course, multiple vacuum sources can be used like the embodiment of FIGS. 3-8.

When the device 200 is placed on the eye in a manner similar to the devices of FIGS. 1-8, a liquid holding chamber 218 is defined by the outer surface of the eye, the upper assembly 217 and the lower assembly 220. Note that a thin gap between glass plate 210 and adapter ring 208 exists to allow air and bubbles expelled during the filling of the liquid holding chamber 218 to escape and to avoid obscuration of the therapeutic laser beam by entrapped bubbles. The proximal surface of the plate 210 is anterior to the proximal surface of the adapter ring 208 so that fluid overflowing the liquid holder chamber 218 during filling will not pool on the glass plate 210 and obscure or divert the therapeutic laser beam.

The forgoing are preferred configurations of the device. Thus, it is understood that the parts of the device may be integral, unitary, composites, fused or combinations of these or other types of materials, as well as combinations of combinations of assemblies and materials, provided that the overall device safely and efficiently accomplishes the objectives of the preferred configuration set forth in FIGS. 3 to 14.

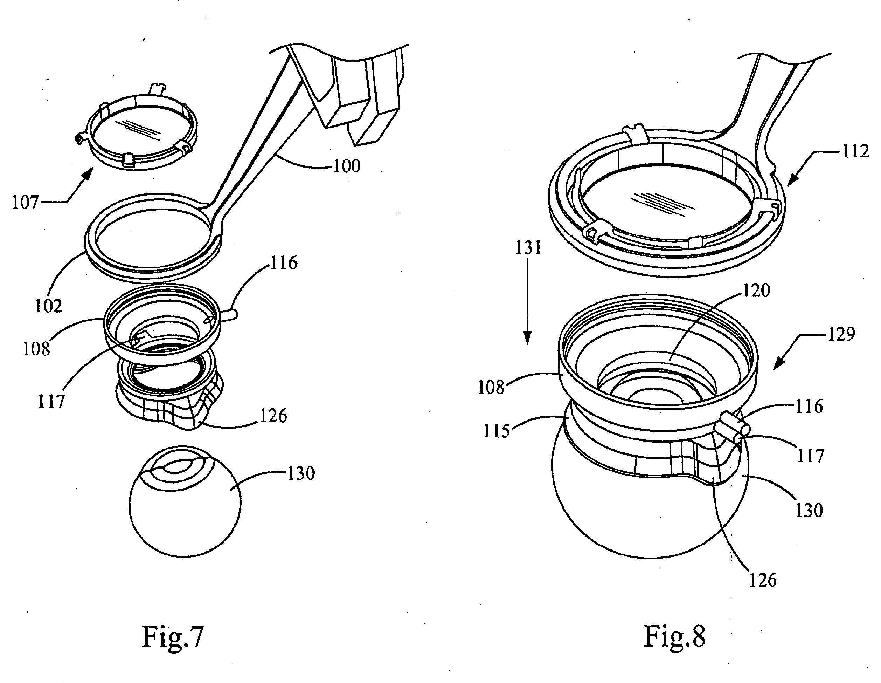

In use the devices of FIGS. 1-14 provide, among other benefits, a novel method of varying, balancing and/or regulating forces that are exerted on the eye during a procedure. In the description to follow, the use of the device of FIGS. 3-8 is similar to the use of the device 200 of FIGS. 9-14 and so the description of the device of FIGS 3-8 that follows applies to the device 200 of FIGS. 9-14. For example, in use the upper assembly 112 of FIG. 8 is attached to the laser device (not shown). The lower assembly 129 is placed and positioned on the eye 130. Once properly positioned on the eye a vacuum is applied by way of vacuum port 117, creating a suction that holds the lower assembly 129 to the eye 130. The amount of vacuum, and by implication the resultant force, that the suction applies to the eye is preferably variable, monitored and controlled. At this initial step the vacuum should be relatively great and at least great enough to ensure that the lower assembly 129 will not shift or become dislodged before the upper assembly 112 is joined to the lower assembly 129. However, care must be taken to avoid damage to the eye and impairment of the patient's ability to fixate. After this initial step the patient is positioned under the upper assembly 112 (alternatively the patient can be positioned under the upper assembly first and then have the lower assembly placed on the eye, the upper assembly can be moved over the patient or combinations of these). The upper assembly 112 is then joined to the lower assembly 129, preferably by lowering upper assembly 112 in the direction of the arrow 131. The upper assembly and in particular the arm 100 of the upper assembly is associated with a means to provide a force, such as a motor that can move the arm in a controlled manner and with a controlled force toward and away from the eye. Once the upper and lower assemblies have been joined a controlled force is then applied to the eye by the device. This force helps to hold the device in place on the eye and prevent movement of the eye. Once this holding force is applied the amount of the vacuum being applied can be reduced. The reducing of this vacuum has benefits to the patient. Further, once the upper and lower assemblies have been joined the device can be filled through port 116 with a liquid.

In this way the forces from the arm and from the vacuum that are placed on the eye during a procedure can be balanced in such a way that minimizes injury to the eye and effect on vision during the procedure. Preferably these forces are balanced so that during the entire duration of the procedure the vision in the eye does not black out The ability to balance and minimize these forces has the further benefit of enabling longer procedure times without damaging the eye; as there is a relationship between the amount of force exerted on the eye and the amount of time the eye is subjected to such force.

Upon completion of the procedure the vacuum is released and preferably the entire device (joined upper and lower assemblies) is moved away from the eye in a direction opposite to that of arrow 131.

From the foregoing description, one skilled in the art can readily ascertain the essential characteristics of this invention, and without departing from the scope thereof, can make various changes and/or modifications of the invention to adapt it to various usages and conditions.

## Claims

1. A self-adjusting liquid holding interface device (1) adapted to be used with a laser surgery apparatus, the device (1) comprising:
a ring shaped structure (2) comprising:
a distal end;
a proximal end, wherein the ring shaped structure (2) has a length defined as the distance between the distal end and the proximal end;
an outer annular structure (3);
an inner annular structure (4), wherein the outer annular structure (3) and the inner annular structure (4) are connected in a telescoping manner;
a vacuum chamber (20) positioned on the distal end for engaging a surface of an eye;
**characterised by** a fluid reservoir (12) filled with a fluid.

2. The device (1) of claim 1, further comprising:
a second vacuum chamber (19) positioned on a distal end of the inner annular structure (4); and
wherein the vacuum chamber (20) is positioned on a distal end of the outer annular structure (3); and
wherein the vacuum chamber (20) and the second vacuum chamber (19) are not in fluid communication with one another so that the device (1) can apply different amounts of vacuum through the first vacuum chamber (20) and the second vacuum chamber (19).

3. The device (1) of claim 1 or claim 2, wherein the fluid has an index of refraction that is approximately the same as an index of refraction of the eye.

4. The device (1) of claim 3, wherein the fluid is saline.

5. The device (1) of claim 1, wherein the fluid reservoir (12) contains an index matching fluid whereby the device (1) eliminates a refractive power of a cornea of the eye.

6. The device (1) of claim 5, wherein the fluid is saline.

7. The device (1) of claim 1, wherein the outer annular structure (3) comprises a second distal end and the inner annular structure (4) comprises a third distal end, wherein the outer structure (3) and the inner structure (4) are configured to have the second distal end and the third distal end move relative to each other and thereby adapt to varying curvatures of the eye; and
a second vacuum chamber (19) positioned on the third distal end for engaging the surface of the eye.

8. The device (1) of claim 7, wherein the vacuum chamber (20) and the second vacuum chamber (19) are not in fluid communication with one another.

9. The device (1) of claim 1, wherein the ring shaped structure (2) is configured to allow a therapeutic laser beam to cut a limbus region of the eye while the device (1) is affixed to the eye by the vacuum chamber.

10. The device (1) of claim 9, wherein the inner annular structure (4) comprises the vacuum chamber and the outer annular structure (3) comprises a second vacuum chamber, wherein the vacuum chamber and the second vacuum chamber are not in fluid communication with each other.

11. The device (1) of claim 1, wherein the outer annular structure (3) comprises a first inner surface and a first outer surface, and the inner annular structure (4) comprises a second inner surface and a second outer surface, wherein the inner annular structure (4) is configured to provide access for a laser beam to at least a portion of a limbus of the eye while the device is engaging the eye; and wherein the outer annular structure (3) and the inner annular structure (4) are adjustably connected so that relative positions of the outer annular structure (3) and the inner annular structure (4) are variable, and wherein the first inner surface is adjacent the second outer surface and the second inner surface is adjacent the first outer surface.

## Patentansprüche

1. Selbstjustierende, flüssigkeitsaufnehmende Verbindungseinrichtung (1), die zur Verwendung mit einer laserchirurgischen Vorrichtung ausgebildet ist, wobei die Einrichtung (1) aufweist:
eine ringförmige Struktur (2), die aufweist:
ein distales Ende,
ein proximales Ende, wobei die ringförmige Struktur (2) eine Länge aufweist, die durch den Abstand zwischen dem distalen Ende und dem proximalen Ende gegeben ist,
eine äußere ringförmige Struktur (3),
eine innere ringförmige Struktur (4), wobei die äußere ringförmige Struktur (3) und die innere ringförmige Struktur (4) in einer teleskopierbaren Weise miteinander verbunden sind,
eine Vakuumkammer (20), die an dem distalen Ende zum Anbringen an einer Oberfläche eines Auges angeordnet ist,
**gekennzeichnet durch** ein Fluidreservoir (12), das mit einem Fluid befüllt ist.

2. Einrichtung (1) nach Anspruch 1, die ferner aufweist:
eine zweite Vakuumkammer (19), die sich an einem distalen Ende der inneren ringförmigen Struktur (4) befindet, und worin die Vakuumkammer (20) an einem distalen Ende der äußeren ringförmigen Struktur (3) angeordnet ist, und worin die Vakuumkammer (20) und die zweite Vakuumkammer (19) miteinander nicht in Fluidverbindung stehen, sodass die Vorrichtung (1) über die erste Vakuumkammer (20) und die zweite Vakuumkammer (19) unterschiedlich starke Vakua anlegen kann.

3. Einrichtung (1) nach Anspruch 1 oder Anspruch 2, worin das Fluid einen Brechungsindex aufweist, der in etwa einem Brechungsindex des Auges entspricht.

4. Einrichtung (1) nach Anspruch 3, worin es sich bei dem Fluid um eine Kochsalzlösung handelt.

5. Einrichtung (1) nach Anspruch 1, worin das Fluidreservoir (12) ein Fluid mit angepasstem Brechungsindex enthält und worin die Einrichtung (1) eine Brechkraft einer Augenhornhaut unterdrückt.

6. Einrichtung (1) nach Anspruch 5, worin es sich bei dem Fluid um eine Kochsalzlösung handelt.

7. Einrichtung (1) nach Anspruch 1, worin die äußere ringförmige Struktur (3) ein zweites distales Ende und die innere ringförmige Struktur (4) ein drittes distales Ende aufweisen, und worin die äußere Struktur (3) und die innere Struktur (4) für eine Bewegung des zweiten distalen Endes und des dritten distalen Endes relativ zueinander ausgebildet sind, um eine Anpassung an unterschiedliche Krümmungen des Auges zu ermöglichen, und
an dem dritten distalen Ende eine zweite Vakuumkammer (19) für ein Anbringen an der Oberfläche des Auges angeordnet ist.

8. Einrichtung (1) nach Anspruch 7, worin die Vakuumkammer (20) und die zweite Vakuumkammer (19) miteinander nicht in Fluidverbindung stehen.

9. Einrichtung (1) nach Anspruch 1, worin die ringförmige Struktur (2) so ausgebildet ist, dass ein therpeutischer Laserstrahl einen Limbusbereich des Auges schneiden kann, wenn die Einrichtung (1) mittels der Vakuumkammer an dem Auge befestigt ist.

10. Einrichtung (1) nach Anspruch 9, worin die innere ringförmige Struktur (4) die Vakuumkammer umfasst und die äußere ringförmige Struktur (3) eine zweite Vakuumkammer umfasst, wobei die Vakuumkammer und die zweite Vakuumkammer miteinander nicht in Fluidverbindung stehen.

11. Einrichtung (1) nach Anspruch 1, worin die äußere ringförmige Struktur (3) eine erste innere Oberfläche und eine erste äußere Oberfläche und die innere ringförmige Struktur (4) eine zweite innere Oberfläche und eine zweite äußere Oberfläche aufweisen, und worin die innere ringförmige Struktur (4) ausgebildet ist, einem Laserstrahl bei Anbringung der Einrichtung an dem Auge einen Zugang zu zumindest einem Teil des Limbus zu ermöglichen, und worin die äußere ringförmige Struktur (3) und die innere ringförmige Struktur (4) einstellbar miteinander verbunden sind, sodass die Lage von äußerer ringförmiger Struktur (3) und innerer ringförmiger Struktur (4) relativ zueinander variabel ist, und worin die erste innere Oberfläche an die zweite äußere Oberfläche und die zweite innere Oberfläche an die erste äußere Oberfläche angrenzen.

## Revendications

1. Dispositif d'interface à réglage automatique contenant un liquide (1) adapté pour être utilisé avec un appareil de chirurgie au laser, le dispositif (1) comprenant :
une structure en forme d'anneau (2) comprenant :
une extrémité distale ;
une extrémité proximale, où la structure en forme d'anneau (2) a une longueur définie comme étant la distance entre l'extrémité distale et l'extrémité proximale ;
une structure annulaire externe (3) ;
une structure annulaire interne (4), où la structure annulaire externe (3) et la structure annulaire interne (4) sont reliées de manière télescopique ;
une chambre à vide (20) positionnée sur l'extrémité distale pour venir en prise avec une surface d'un oeil ;
**caractérisé par** un réservoir de fluide (12) rempli de fluide.

2. Dispositif (1) de la revendication 1, comprenant en outre :
une deuxième chambre à vide (19) positionnée sur une extrémité distale de la structure annulaire interne (4) ; et
dans lequel la chambre à vide (20) est positionnée sur une extrémité distale de la structure annulaire externe (3) ; et
dans lequel la chambre à vide (20) et la deuxième chambre à vide (19) ne sont pas en communication fluidique l'une avec l'autre de sorte que le dispositif (1) puisse appliquer différentes quantités de vide à travers la première chambre à vide (20) et la deuxième chambre à vide (19).

3. Dispositif (1) de la revendication 1 ou de la revendication 2, dans lequel le fluide a un indice de réfraction qui est approximativement le même qu'un indice de réfraction de l'oeil.

4. Dispositif (1) de la revendication 3, dans lequel le fluide est une solution saline.

5. Dispositif (1) de la revendication 1, dans lequel le réservoir de fluide (12) contient un fluide d'adaptation d'indice moyennant quoi le dispositif (1) supprime un pouvoir de réfraction d'une cornée de l'oeil.

6. Dispositif (1) de la revendication 5, dans lequel le fluide est une solution saline.

7. Dispositif (1) de la revendication 1, dans lequel la structure annulaire externe (3) comprend une deuxième extrémité distale et la structure annulaire interne (4) comprend une troisième extrémité distale, dans lequel la structure externe (3) et la structure interne (4) sont configurées pour amener la deuxième extrémité distale et la troisième extrémité distale à se déplacer l'une par rapport à l'autre et pour s'adapter ainsi à des courbures variables de l'oeil ; et
une deuxième chambre à vide (19) positionnée sur la troisième extrémité distale pour venir en prise avec la surface de l'oeil.

8. Dispositif (1) de la revendication 7, dans lequel la chambre à vide (20) et la deuxième chambre à vide (19) ne sont pas en communication fluidique l'une avec l'autre.

9. Dispositif (1) de la revendication 1, dans lequel la structure en forme d'anneau (2) est configurée pour permettre à un faisceau laser thérapeutique de découper une région de limbe de l'oeil tandis que le dispositif (1) est fixé à l'oeil par la chambre à vide.

10. Dispositif (1) de la revendication 9, dans lequel la structure annulaire interne (4) comprend la chambre à vide et la structure annulaire externe (3) comprend une deuxième chambre à vide, dans lequel la chambre à vide et la deuxième chambre à vide ne sont pas en communication fluidique l'une avec l'autre.

11. Dispositif (1) de la revendication 1, dans lequel la structure annulaire externe (3) comprend une première surface interne et une première surface externe, et la structure annulaire interne (4) comprend une deuxième surface interne et une deuxième surface externe, dans lequel la structure annulaire interne (4) est configurée pour fournir l'accès d'un faisceau laser à au moins une partie d'un limbe de l'oeil tandis que le dispositif est en prise avec l'oeil ; et dans lequel la structure annulaire externe (3) et la structure annulaire interne (4) sont reliées de manière réglable de sorte que des positions relatives de la structure annulaire externe (3) et de la structure annulaire interne (4) soient variables, et dans lequel la première surface interne est adjacente à la deuxième surface externe et la deuxième surface interne est adjacente à la première surface externe.
